# EUROPÄISCHE PATENTSCHRIFT

(11) **EP 0 912 751 B1**
(45) Veröffentlichungstag und Bekanntmachung des Hinweises auf die Patenterteilung: **04.10.2000**
(21) Anmeldenummer: 97932775.6
(22) Anmeldetag: 04.07.1997
(51) Int. Cl.: C12P 17/10, C12N 1/00, C12N 1/20, C12N 9/78

(54) **VERFAHREN ZUR HERSTELLUNG VON D-PROLINDERIVATEN MITTELS MIKROORGANISMEN**
PROCESS FOR PREPARATION OF D-PROLINE DERIVATIVES BY MEANS OF MICRO-ORGANISMS
PROCEDE DE FABRICATION DE DERIVES DE D-PROLINE AU MOYEN DE MICRO-ORGANISMES

(30) Priorität: 08.07.1996 CH 169696
(43) Veröffentlichungstag der Anmeldung: 06.05.1999
(73) Patentinhaber: LONZA AG, CH-3945 Gampel/Wallis (CH)
(72) Erfinder: BERNEGGER, Christine, CH-3985 Münster (CH); BRUX, Frank, CH-3942 Raron (CH); GOSTELI, Jacques, CH-4059 Basel (CH)
(74) Vertreter: Winter, Brandl & Partner
(86) Internationale Anmeldenummer: EP9703546
(87) Internationale Veröffentlichungsnummer: WO9801577

(56) Entgegenhaltungen:
- US-A- 3 806 420
- DATABASE WPI Section Ch, Week 9414 Derwent Publications Ltd., London, GB; Class B03, AN 94-114270 XP002043866 & JP 06 062 880 A (KYOWA HAKKO KOGYO KK) , 8.März 1994

## Beschreibung

Die Erfindung betrifft ein neues Enzym mit Amidinohydrolase-Aktivität, neue Mikroorganismen, die diese Amidinohydrolasen enthalten, sowie ein neues Verfahren zur Herstellung von D-Prolinderivaten unter Verwendung der Mikroorganismen bzw. Amidinohydrolasen.

D-Prolinderivate sind wichtige Zwischenprodukte zur Herstellung von Pharmazeutika (J. Org. Chem., 1994, 59, 7496 - 7498).

Die Herstellung von Aminosäuren und Aminosäurederivaten mittels Mikroorganismen oder Enzymen ist bekannt. So wird in der US-A-3 806 420 die Herstellung von Sarkosin durch Harnstoffabspaltung aus Kreatinin unter Verwendung einer Amidinohydrolase aus beispielsweise Pseudomonas beschrieben.
Die JP-A-6062880 beschreibt die Herstellung von 4-Hydroxy-L-prolin aus 4-Hydroxy-L-ornithin mit Mikroorganismen beispielsweise der Gattungen Pseudomonas, Agrobacterium oder Arthrobacter mittels enzymatischer Zyklisierung.

Auch zur Herstellung von D-Prolin sind mehrere Verfahren bekannt.
Die JP-A 92183399 beschreibt ein Verfahren zur Herstellung von D-Prolin ausgehend von (DL)-Prolin mittels Mikroorganismen der Gattung Candida oder Trichospora. Dieses Verfahren hat den Nachteil, dass die Umsetzungszeit zu lang ist und D-Prolin in geringer Ausbeute erhalten wird.
Die JP-A 07127354 beschreibt ein Verfahren zur Herstellung von D-Prolin ausgehend von Ornithin mittels Mikroorganismen der Spezies Proteus mitajiri. Nachteilig bei diesem Verfahren ist, dass zum einen das Edukt Ornithin zu kostspielig ist zum anderen, dass D-Prolin in schlechter Ausbeute erhalten wird.
Desweiteren beschreibt die JP-A 07289275 ein Verfahren zur Herstellung von D-Prolin ausgehend von L-Prolin. Dabei wird L-Prolin mittels Mikroorganismen der Gattung Escherichia, welche Racemase-Aktivität besitzen, zu (DL)-Prolin racemisiert, welches dann mit L-Prolin abbauenden Mikroorganismen kultiviert wird, um D-Prolin zu erhalten. Nachteilig bei diesem Verfahren ist, dass das dabei erhaltene D-Prolin noch weiter gereinigt werden muss und dies mit hohen Ausbeuteverlusten verbunden ist.

Aufgabe der vorliegenden Erfindung ist es, eine Amidinohydrolase bzw. Mikroorganismen, die dieses Enzym enthalten, zur Verfügung zu stellen, die für ein kostengünstiges Verfahren zur Herstellung von D-Prolinderivaten eingesetzt werden können, bei dem D-Prolin in guter Ausbeute erhalten wird.
Diese Aufgabe wird mit den Mikroorganismen gemäss Anspruch 1, der Amidinohydrolase gemäss Anspruch 4, sowie mit dem Verfahren gemäss Anspruch 5 gelöst.

Die erfindungsgemässen Mikroorganismen können aus Bodenproben, Schlamm oder Abwasser unter Zuhilfenahme üblicher mikrobiologischer Techniken isoliert werden. Erfindungsgemäss erfolgt die Isolation der Mikroorganismen derart, dass man diese in einem Medium enthaltend ein Guanidinvaleriansäurederivat der allgemeinen Formel 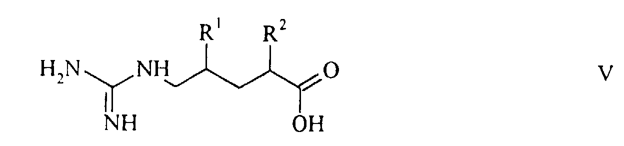 worin R¹ Wasserstoff oder Hydroxy und R² ein Halogenatom oder -NH₂ bedeuten, als einzige Stickstoffquelle mit einer geeigneten Kohlenstoffquelle auf übliche Weise züchtet. Als Guanidinvaleriansaurederivate der allgemeinen Formel V sind z. B geeignet: L-α-Chlor-δ-guanidinvaleriansäure, D-α-Chlor-δ-guanidinvaleriansäure, (DL)-α-Chlor-δ-guanidinvaleriansäure, L-α-Brom-δ-guanidinvaleriansäure, D-α-Brom-δ-guanidinvaleriansäure, (DL)-α -Brom-δ-guanidinvaleriansäure, L-α-Chlor-γ-hydroxy-δ-guanidinvaleriansäure, D-α-Brom-γ-hydroxy-δ-guanidinvaleriansäure, L-, D- oder (DL)-Arginin.

Aus der durch Züchtung erhaltenen Kultur werden dann zweckmässig jene Mikroorganismen selektioniert, die L-α-Chlor-δ-guanidinvaleriansäure (R¹ = H, R² = Cl) oder L-Arginin (R¹ = H, R² = NH₂) als einzige Stickstoffquelle verwerten.

Als geeignete Kohlenstoffquelle können die Mikroorganismen beispielsweise Zucker, Zuckeralkohole oder Dicarbonsäuren als Wachstumssubstrat nützen.

Als Zucker können Hexosen wie Glucose verwendet werden. Als Zuckeralkohole kann beispielsweise Glycerin Verwendung finden. Als Dicarbonsäure kann z. B. Succinat verwendet werden. Desweiteren kann als Kohlenstoffquelle Arginin, Agmatin (4-Aminobutylguanin), Glutamin oder Glutaminsäure eingesetzt werden.

Als Selektions- und Anzuchtmedium können die in der Fachwelt üblichen verwendet werden, wie beispielsweise das in Tabelle 1 beschriebene. Vorzugsweise wird das in Tabelle 1 beschriebene verwendet.

Während der Anzucht und Selektion werden zweckmässig die wirksamen Enzyme der Mikroorganismen induziert Als Enzyminduktor kann beispielsweise α-Chlor-δ-guanidinvaleriansäure, L-Arginin, Guanidin, Guanidinhydrochlorid oder Guanidinacetat verwendet werden. Die Induktion der wirksamen Enzyme kann jedoch auch in einem Vollmedium wie bspw. mit "Nutrient Yeast Broth" (NYB) erfolgen, wenn einer der Induktoren darin enthalten ist.

Üblicherweise erfolgt die Anzucht und Selektion bei einer Temperatur von 20 bis 40 °C, vorzugsweise von 25 bis 40 °C und bei einem pH-Wert zwischen pH 5 und pH 9,5 vorzugsweise zwischen pH 8 und pH 9,5.

Bevorzugt werden als Mikroorganismen α-Chlor-δ-guanidinvaleriansäure-verwertende der Gattung Pseudomonas, Arthrobacter, Agrobacterium oder Klebsiella, insbesondere Mikroorganismen der Spezies Agrobacterium radiobacter, Pseudomonas cepacia, Klebsiella pneumoniae DSM 10593, Pseudomonas aeruginosa DSM 10581 oder Arthrobacter sp. DSM 10582 , sowie deren funktionell aequivalente Varianten und Mutanten, isoliert. Die Stämme Klebsiella pneumoniae, Arthrobacter sp. und Pseudomonas aeruginosa wurden am 11.03.1996 bei der Deutschen Sammlung von Mikroorganismen und Zellkulturen GmbH, Mascherodenveg 1b, D-38124 Braunschweig gemäss Budapester Vertrag hinterlegt.

Unter "funktionell aequivalenten Varianten und Mutanten" werden Mikroorganismen verstanden, die im wesentlichen dieselben Eigenschaften und Funktionen wie die Ursprungsmikroorganismen besitzen. Derartige Varianten und Mutanten können zufällig, z. B. durch UV-Bestrahlung oder durch mutagene Chemikalien gebildet werden.

### Taxonomische Beschreibung von Klebsiella pneumoniae (DSM 10593)

### Eigenschaften des Stammes

| | | | |
|---|---|---|---|
| Zellform | Stäbchen | ADH | - |
| Breite µm | 1,0 - 1,2 | LDC | - |
| Länge µm | 1,2 - 2,0 | ODC | - |
| Beweglichkeit | - | VP | + |
| Gram-Reaktion | - | Indol | - |
| Lyse durch 3% KOH | + | H₂S-Bildung | - |
| Aminopeptidase (Cerny) | + | Simmons Citrat | + |
| Sporen | - | Urease | + |
| Oxidase | - | Methylrot | - |
| Catalase | + | Hydrolyse von | |
| Wachstum anaerob | + | Gelatine | - |
| Gas aud Glucose | + | DNA | - |
| Säure aus (ASS) | | Tween 80 | - |
| Glucose | + | | |
| Fructose | + | **Abkürzungen:** | |
| Xylose | + | ASS: Acetylsalicylsäure | |
| Erythrit | - | ONPG: o-Nitro-phenylgalactosidase | |
| Adonit | - | ADH: Alkoholdehydrogenase | |
| D-Mannose | + | LDC: Lactatdecarboxylase | |
| L-Rhamnose | + | ODC: Ornithindecarboxylase | |
| Inosit | + | VP: Voges Proskauer | |
| Sorbit | + | | |
| α-Methyl-D-glucosid | + | | |
| Cellobiose | + | | |
| Maltose | + | | |
| Lactose | + | | |
| D-Arabitol | + | | |
| ONPG | + | | |

### Taxonomische Beschreibung von Arthrobacter sp. (DSM 10582)

Charakterisierung Gram-positive, coryneforme Stäbchen, in älteren Kulturen kockoid; strickt aerob; keine Säure- oder Gasbildung aus Glucose

| | |
|---|---|
| Beweglichkeit | - |
| Sporen | - |
| Katalase | + |

*meso*-Diaminopimelinsäure in der Zellwand: nein
Peptidoglycan-Typ: nb

Die erfindungsgemässe Amidinohydrolase ist erhältlich aus den zuvor beschriebenen Mikroorganismen und befähigt von Guanidinvaleriansäurederivaten, ausgewählt aus Verbindungen der allgemeinen Formel V, Harnstoff abzuspalten.

Zweckmässig wird die Amidinohydrolase aus den Mikroorganismen der Gattung Klebsiella, Pseudomonas, Agrobacterium oder Arthrobacter, insbesondere aus den Mikroorganismen der Spezies Agrobacterium radiobacter, Pseudomonas cepacia, Klebsiella pneumoniae DSM 10593. Arthrobacter sp DSM 10582 oder Pseudomonas aeruginosa DSM 10581 gewonnen.

Zur Gewinnung der erfindungsgemässen Amidinohydrolase werden diese Mikroorganismen in einem wässrigen Nährmedium, das eine Kohlenstoff-, Stickstoffquelle, Mineralsalze und eine Vitaminquelle enthält, auf übliche Weise gezüchtet (kultiviert). Zweckmässig werden die Mikroorganismen bei einer Temperatur von 20 bis 40 °C und bei einem pH-Wert von 5 bis 8 kultiviert. Die Amidinohydrolasen können dann nach Aufschluss der Mikroorganismen-Zellen wie z. B. durch Ultraschall-, French-Press oder Lysozym-Methode durch an sich bekannte Methoden der Enzymreinigung isoliert werden.

Zweckmassig besitzt die Amidinohydrolase folgende Eigenschaften:
a) ein pH-Optimum von pH 8,5 ± 1,
b) ein Temperaturoptimum von ca. 37 °C bei einem pH zwischen 7 und 8,
c) einen K_{M}-Wert für das Substrat L-α-Chlor-δ-guanidinvaleriansäure von 85,2 mM ± 5 (100 mM Phosphatpuffer; 37 °C),
ferner die Eigenschaft, dass sie
d) durch L-α-Chlor-δ-guanidinvaleriansäure, L-Arginin, Guanidinhydrochlorid, Guanidin und / oder Guanidinacetat induziert wird,
e) gehemmt wird von L-α-Chlor-δ-guanidinvaleriansäure und
f) die Substrate Arginin, L-α-Chlor-δ-guanidinvaleriansäure und L-α-Brom-δ-guanidinvaleriansäure zum entsprechenden L-Aminovaleriansäurederivat hydrolysiert.

Zur erfindungsgemässen Herstellung von D-Prolinderivaten wird zweckmäßig in einer ersten Verfahrensstufe ein L-Argininderivat, oder dessen Salz, der allgemeinen Formel 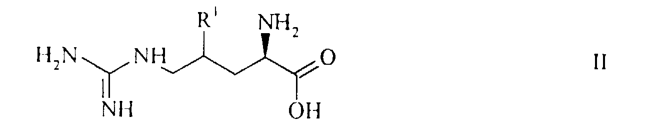 worin R¹ die genannte Bedeutung hat, auf an sich bekannte Weise in ein L-Guanidinvaleriansäurederivat, oder dessen Salz, der allgemeinen Formel 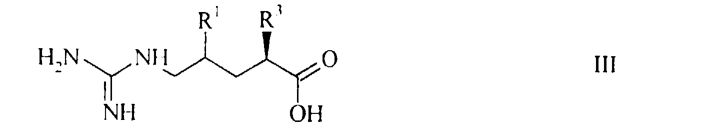 worin R¹ die genannte Bedeutung hat und R³ ein Halogenatom bedeutet, überführt. Dabei wird als Zwischenprodukt das entsprechende Diazoniumsalz gebildet.

Als L-Argininderivat kann L-Arginin oder L-γ-Hydroxy-Arginin venvendet werden.

Als Salze des L-Argininderivats und des L-Guanidinvaleriansäuredervats können deren Hydrochloride oder Hydrobromide eingesetzt werden.

Üblicherweise wird die Diazotierung in der ersten Stufe mit einer Nitritlösung oder mit Salpetersäure durchgeführt. Als Nitritlösung kann eine Natrium- oder Kaliumnitritlösung venvendet werden. Vorzugsweise wird Salpetersäure, insbesondere 50 bis 65 %ige Salpetersäure, verwendet.

Die Umsetzung in der ersten Stufe wird üblicherweise in Gegenwart einer Halogenwasserstoffsäure durchgeführt. Als Halogenwasserstoffsäure kann Salzsäure oder Bromwasserstoffsäure verwendet werden, vorzugsweise wird Salzsäure verwendet.

Zweckmässig wird die Umsetzung in der ersten Stufe bei einer Temperatur von -10 °C bis 100 °C, vorzugsweise von 0 °C bis 80 °C durchgeführt.

In der erfindungsgemäßen zweiten Verfahrenstufe wird das L-Guanidinvaleriansäurederivat (Formel III) mittels einer Amidinohydrolase gemäss Anspruch 4, mittels Miroorganismen gemäss Anspruch 1 und/oder Enzymextrakten daraus in ein L-Aminovaleriansäurederivat der allgemeinen Formel 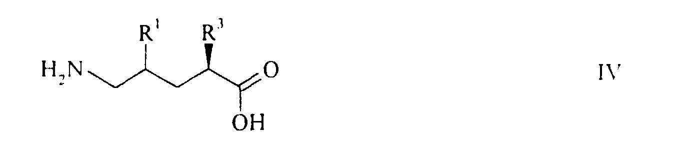 worin R¹ und R³ die genannte Bedeutung haben, hydrolysiert.

Prinzipiell ist die mikrobiologische Hydrolyse sowohl mit den bereits beschriebenen Amidinohydrolasen als auch mit den bereits beschriebenen Mikroorganismen und Enzymextrakten möglich. Für die Hydrolyse besonders geeignet sind die zuvor beschriebenen Mikroorganismen der Gattung Pseudomonas, Arthrobacter, Agrobacterium oder Klebsiella , insbesondere die Mikroorganismen der Spezies Klebsiella pneumoniae DSM 10593, Pseudomonas aeruginosa DSM 10581, Arthrobacter sp. DSM 10582, Agrobacterium radiobacter oder Pseudomonas cepacia, sowie deren funktionell aequivalente Varianten und Mutanten.

Die mikrobiologische Hydrolyse kann nach üblichem Anzüchten der Mikroorganismen mit ruhenden Zellen (nicht wachsende Zellen, die keine Kohlenstoff- und Energiequelle mehr benötigen) oder mit wachsenden Zellen durchgeführt werden. Vorzugsweise wird die Hydrolyse mit ruhenden Zellen durchgeführt.

Für die mikrobiologische Hydrolyse können fachmännisch übliche Medien eingesetzt werden wie beispielsweise niedermolare Phosphat- und Hepes-Puffer, Vollmedien wie z. B. "Nutrient Yeast Broth" (NYB) oder das in Tabelle 3 beschriebene Medium. Vorzugsweise wird die Hydrolyse entweder in einem niedermolaren Phosphat- oder Hepes-Puffer durchgeführt.

Zweckmässig wird die Hydrolyse unter einmaliger oder kontinuierlicher Zugabe von dem L-Guanidinvaleriansäurederivat so durchgeführt, dass die Konzentration 20 Gew.%, vorzugsweise 1 Gew.%, nicht übersteigt.

In der dritten Stufe wird das L-Aminovaleriansäurederivat (allgemeine Formel IV) zum Endprodukt der allgemeinen Formel 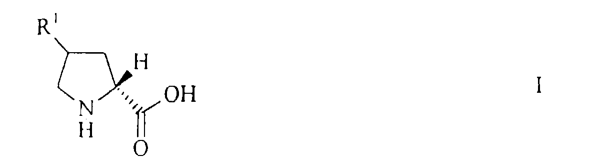 worin R¹ die genannte Bedeutung hat, cyclisiert.

Zweckmässig wird der pH-Bereich des Mediums so gewählt, dass die zweite Stufe und die dritte Stufe ohne Isolation des L-Aminovaleriansäurederivats (Formel IV) durchgeführt wird. Bei dieser bevorzugten Ausführungsform wird der pH-Wert des Mediums auf pH 5 bis pH 13, vorzugsweise auf pH 7 bis pH 9,5, eingestellt. In diesem Bereich erfolgt die Cyclisierung von dein L-Aminovaleriansäurederivat (Formel IV) zum D-Prolin spontan Zweckmässig erfolgt die Cyclisierung und die Hydrolyse bei einer Temperatur von 10 bis 60 °C, vorzugsweise von 30 bis 40 °C.

Grundsätzlich kann die Herstellung der D-Prolinderivate der Formel I auch dadurch erfolgen, daß man das L-Argininderivat der Formel II, oder dessen Salze, unmittelbar mit den oben beschriebenen Mikroorganismen, Enzymextrakten oder Amidinohydrolasen zu Verbindungen der Formel VI hydrolysiert, 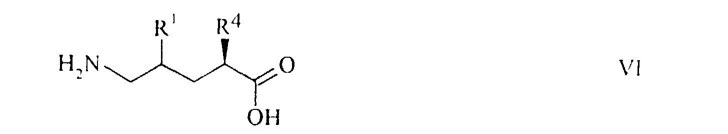 worin R¹ die genannte Bedeutung hat und R⁴ -NH₂ bedeutet, diese Verbindungen anschließend wie oben beschrieben über die Zwischenstufe eines Diazoniumsalzes in ein L-Aminovaleriansäurederivat der Formel IV überführt und letzteres zu den gewünschten Prolinderivaten cyclisiert. Dieses Verfahren ist jedoch weniger bevorzugt.

### Beispiele:

### Beispiel 1

### Isolation der Mikroorganismen Klebsiella pneumoniae, Pseudomonas cepacia, Agrobacterium radiobacter und Arthrobacter sp.

Erdproben aus Gartenkompost oder der Kläranlage LONZA (Visp) wurden in dem folgenden Minimalmedium bei 37 °C unter Schütteln inkubiert.

Glucose oder Glycerin wurden in Konzentrationen von 1 - 20 g/l als Kohlenstoffquelle angeboten.

Als Stickstoffquellen und/oder Induktoren wurden L-Arginin, Harnstoff, (NH₄)₂SO₄, L-α-Chlor-δ-guanidinvaleriansäure in Konzentrationen von 0,25 - 20 mM eingesetzt.

Hefeextrakt wurde in Konzentrationen von 0,1 - 1 g/l als Supplement zugegeben.

### A) Flüssigmedium

**Tabelle 1:**

| | |
|---|---|
| MgCl₂ x 6H₂O | 0,4 g/l |
| CaCl₂ x 2H₂O | 0,014 g/l |
| FeCl₃ x 6H₂O | 2,8 mg/l |
| Na₂SO₄ | 0,1 g/l |
| Na₂HPO₄ | 2 g/l |
| KH₂PO₄ | 1 g/l |
| NaCl | 2 g/l |
| Vitaminlösung | 1 ml /l |
| Spurenelementlösung | 1 ml /l |
| pH 6,8 - 8,0 | |

### B) Festmedien

Zu A) zusatzlich 20 g/l Agar.

Mikroorganismen wuchsen über eine Inkubationszeit von 2 - 30 Tagen auf den entsprechenden Medien.

Sie wurden 1 - 3mal auf frische Medien überimpft und auf Festmedien vereinzelt.

Zellsuspensionen der isolierten Mikroorganismen wurden auf die Aktivität untersucht (vgl. Beispiel 3). Folgende Mikroorganismen wurden isoliert: Klebsiella pneumoniae (DSM 10293), Arthrobacter sp. (DSM 10582), Pseudomonas cepacia und Agrobacterium radiobacter.

### Beispiel 2:

### Selektion von Spontanmutanten

Der Wildtyp Pseudomonas aeruginosa (P aeruginosa) PAO1 (Holloway, B. W., Bacteriol. Rev., 1959, 33, 419 - 443) wurde auf Fest- oder Flüssigmedium wie in Beispiel 1 beschrieben mit L-α-Chlor-δ-guanidinvaleriansäure als einzige Stickstoffquelle und Glycerin oder Glucose als Kohlenstoffquelle 1 - 30 Tage bei 37 °C inkubiert. Nach dieser Zeit wuchsen auf den Fest medien 11 gleiche, leicht bräunliche Kolonien. Die Einzelkolonien wurden abermals ausgestrichen, und dann auf entsprechendem flüssigen Minimalmedium gezüchtet. Zellsuspensionen solcher Zellen wurden auf die entsprechende Enzymaktivität hin untersucht. Auf diese Weise wurde ein Pseudomonas aeruginosa (DSM 10581) isoliert, der eine Hydrolase nach gewünschter Art exprimierte. Nach "Phagen-typing" und Stammidentifikation mit API-Test(20 NE bio Merieux SA, France) war der Stamm mit dem Wildtyp Pseudomonas aeruginosa PAO1 noch identisch, er unterscheidet sich im Wesentlichen vom Wildtyp durch seine Fähigkeit, eine L-α-Chlor-δ-guanidinvaleriansäure Amidinohydrolase zu exprimieren.

### Beispiel 3:

### Induktion und Biotransformation mit ruhenden Zellen mit den Bakterienstämmen Klebsiella pneumoniae DSM 10593 und Pseudomonas aeruginosa DSM 10581

Die in Beispiel 1 und 2 beschrieben isolierten Mikroorganismen wurden auf 100 ml Flüssigmedium A) mit Glycerin oder Glucose als Kohlenstoffquelle und L-α-Chlor-δ-guanidinvaleriansäure als Induktor und Stickstoffquelle bei einer Temperatur von 37 °C gezüchtet. Nach Erreichen der stationären Wachstumsphase (OD650 von 0,8 - 1,5) wurden die Zellen durch Zentrifugation geerntet und 10 - 20mal konzentrierter in die Biotransformationslösung aufgenommen. Die Biotransformationslösung hatte folgende Zusammensetzung: 10 - 100 mM Phosphat oder Hepes-puffer, 10 - 150 mM α-Chlor-δ-guanidinvaleriansäure, pH 6,8 - 11). Die Biotransformtion wurde bei 37 °C unter leichtem Schütteln durchgeführt. Proben wurden in Zeitintervallen entnommen und durch Dünnschichtchromatographie oder HPLC analysiert.

L-α-Chlor-δ-guanidinvaleriansäure wurde dabei je nach Konzentration in einer Zeitspanne von 5 - 24 h quantitativ in D-Prolin übergeführt, wobei der ee-Wert von D-Prolin über 96% lag (nach HPLC).

### Beispiel 4:

### Induktion und Biotransformation mit Bakterienstamm Arthrobacter sp. DSM 10582

Die Anzucht auf folgenden Medien erlaubte die Induktion der Amidinohydrolase: NYB (Difco); NYB und 1 - 10 mM L-α-Chlor-δ-guanidinvaleriansäure; Medium (A) mit Glycerin 25 mM und L-Arginin 1 - 10 mM als Stickstoffquelle. Zellen wurden über 24 h bei 37 °C in 100 ml Medium gezüchtet. Zellen wurden wie in Beispiel 3 beschrieben geerntet und für die Biotransformation verwendet. Nach Waschen der Zellen wurde die Biotransformation in Phosphat- oder Hepespuffer 10 - 200 mM durchgeführt (pH 5 - 10). L-α-Chlor-δ-guanidinvaleriansäure wurde dabei je nach Konzentration über eine Zeitspanne von 5 - 24 h quantitativ in D-Prolin übergeführt, wobei der ee-Wert von D-Prolin über 96% lag (nach HPLC).

### Beispiel 5:

### Induktion und Biotransformation mit Pseudomonas aeruginosa DSM 10581

Der Stamm wurde in 100 ml Erlenmeyer-Kolben mit Schikane bei 37 °C unter Schütteln auf folgenden Medien gezüchtet: Minimalmedium (A) mit den wie in nachfolgender Tabelle 2 beschriebenen Zusätzen, sowie NYB-Vollmedium enthaltend Trypton 10 g/l, "Meat extract" 5 g/l, NaCl 5 g/l). Die Zellen wurden wie in Beispiel 3 beschrieben für die Biotransformation vorbereitet.

**Tabelle 2**

| C-Quelle mM | N-Quelle mM | Induktor mM | Hefeextrakt g/l | OD650 24 h | Biotransformations-Rate % |
|---|---|---|---|---|---|
| | | | | | |
| Gluc 20 | - | Cl-Arg 2 | 0,2 | - | 25 |
| Gluc 20 | Arg 1 | Cl-Arg 4 | | 1,7 | 100 |
| Gluc 20 | Arg 1 | Cl-Arg 3 | 0,2 | - | 25 |
| Gluc 20 | NH₄⁺ 1 | Cl-Arg 2 | 0,2 | - | 25 |
| Gluc 20 | NH₄⁺ 1 | Cl-Arg 4 | 0,2 | - | 25 |
| Gluc 20 | Arg 2 | Cl-Arg 2 | 0,1 | 1,6 | 30 |
| Gluc 20 | Ornit 1 | Cl-Arg 1 | 0,1 | 1 | 10 |
| Gluc 20 | Citrul 1 | Cl-Arg 1 | 0,1 | 0,9 | 6 |
| Gly 20 | Arg 2 | Cl-Arg 2 | 0,1 | 1 | 30 |
| Gly 20 | Ornit 1 | Cl-Arg 1 | 0,1 | 0,9 | 20 |
| Gly 20 | Citrul 1 | Cl-Arg 1 | 0,1 | 0,75 | 30 |
| Gly 10 | Arg 10 | Cl-Arg 0,5 | 0,1 | | 30 |
| Gluc 20 | Guanin 5 | - | | 0,4 | 23 |
| Gluc 20 | Guanin 5 | Arginin 1 | | 1,1 | 20 |
| Gluc 20 | Guanidinacetat 5 | - | | 0,5 | 20 |

| C-Quelle mM | N-Quelle mM | Induktor mM | OD 24h | Biotransformations-Rate % | |
|---|---|---|---|---|---|
| | | | | | |
| Glu 10 | - | Cl-Arg 5 | 0,6 | 10 | |
| Glu 20 | - | Cl-Arg 5 | 0,8 | 30 | |
| Gluc 20 | Glu 10 | Cl-Arg 5 | 1,2 | 70 | |
| Gln 10 | - | Cl-Arg 5 | 0,3 | 10 | |
| Gln 20 | - | Cl-Arg 5 | 0,5 | 10 | |
| Gluc 10 | Betain 10 | Cl-Arg 5 | 1,2 | 70 | |
| Arg 10 | - | Cl-Arg 5 | 0,5 | 70 | |
| Gluc 10 | Arg 1 | Cl-Arg 5 | 0,8 | 70 | |
| Agmatin 10 | - | Cl-Arg 5 | 0,2 | 20 | |
| Gluc = Glucose, Arg = Arginin, Gly = Glycerin , Glu = Glutaminsäure, Gln = Glutamin Cl-Arg = L-α-Chlor-δ-guanidinvaleriansäure, Ornit = Ornithin, Citrul = Citrulin | | | | | |

L-α-Chlor-δ-guanidinvaleriansäure wurde dabei je nach Konzentration in einer Zeitspanne von 5 - 24 Stunden quantitativ in D-Prolin übergeführt, wobei der ee-Wert von D-Prolin über 96% lag (nach HPLC). Auf diese Weise entstanden 13 g/l D-Prolin (nach HPCL). Die besten Resultate wurden in dem genannten Medium mit den Zusätzen Gluc 20 mM, Arg 1 mM und Cl-Arg 4 mM (vgl. Tabelle 2) erreicht.

### Beispiel 6:

### Herstellung von L-α-Chlor-δ-guanidinvaleriansäure

Hierzu wurden 100 g (0,475 mol) L-Argininmonohydrochlorid in 150 ml konzentrierter Salzsäure gelöst und auf 65 °C erwärmt. Innerhalb von 30 Minuten wurden 75 ml 65%igen HNO₃ zugetropft, die Zugabe war von Beginn an von einer heftigen Gasentwicklung begleitet, nach weiteren 30 Minuten bei 65 °C war die Gasentwicklung beendet und die klare, leicht gelbliche Reaktionslösung wurde im Vakuum eingeengt. Der erhaltene Rückstand wurde noch zweimal in je 200 ml konzentrierter HCl aufgenommen und im Vakuum bis zur Trockene eingeengt. Man erhielt 112,2 g eines leicht gelblichen Feststoffes. Der Feststoff wurde in 750 ml konzentrierter HCl bei 60 °C aufgelöst und durch Abkühlen der Lösung bis 0 °C kristallisiert. Der Niederschlag wurde abfiltriert, zweimal mit je 100 ml kalter 6 N HCl gewaschen und im Vakuum getrocknet. Man erhielt 72,9 g eines farblosen, kristallinen Feststoffs, entsprechend einer Ausbeute von 66,7%.
Smp. 149 °C
α_{D}²⁵(c=10% in H₂O) = -7,87°
¹H-NMR in ppm (400 MHz, in D₂O): 4,55 (dd, 1H, H-1); 3,25(t, 2H, H-4); 2,15- 1,95 (br. m, 2H); 1,8 - 1,7 (br. m, 2H).
Gehalt: 100,8%

### Beispiel 7

### a) Herstellung von D-Prolin im 3,5 l Fermenter

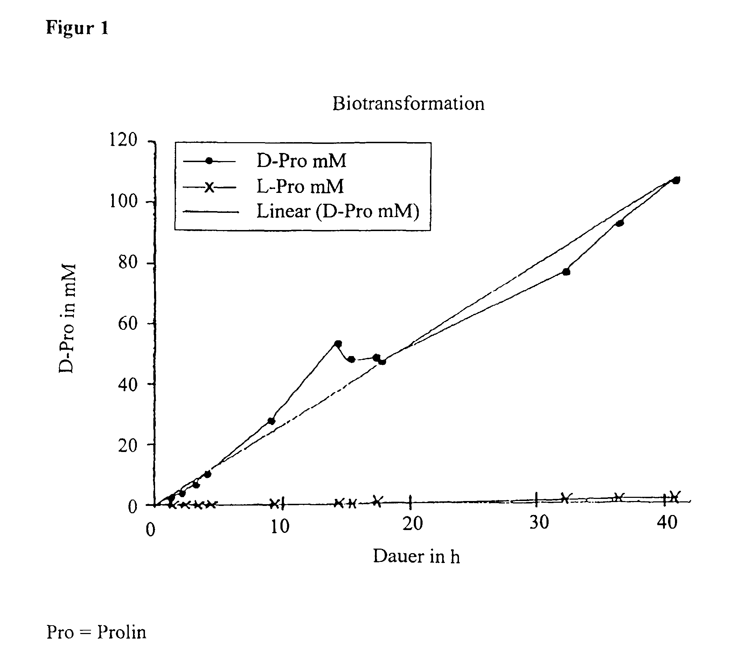

Zellen des Stammes Pseudomonas aeruginosa (DSM 10581) wurden mit dem in Tabelle 3 beschriebenen Medium mit 10% Vorkultur beimpft und in 24 h bis zu einer OD₆₀₀ von 13 angezüchtet. Die Biomasse wurde durch Zentrifugation abgetrennt, in 10 mM Hepes pH 8,5 gewaschen und in diesem resuspendiert. Die Biotransformation erfolgte in einem 11 Applikonfermentor, unter pH-Kontrolle bei einem konstanten Wert von pH 8,5, bei 37 °C, die Zelldichte betrug bei OD₆₀₀ 10-20. Die Konzentration von α-Chlor-δ-guanidinovaleriansäure lag während der Biotransformation immer über 25 mM. Insgesamt wurden 110 mM Substrat zugegeben. Nach 40h war 106 mM. D-Prolin mit einem ee-Wert von 98,3% (nach HPLC) gebildet (Figur 1).

Nach 40 h wurden die Zellen mittels Zentrifugation abgetrennt. Die Rohlösung wurde über Celite 535 gereinigt; nach einer Proteinfällung mit Perchlorsäure und anschliessender Neutralisation mit KOH wurde die Biotransformationslösung einer Aktivkohlebehandlung unterzogen, und über Celite 535 abfiltriert. Das Produkt wurde zur Trockene eingeengt.

### b) Herstellung von D-Prolin mit zellfreiem Extrakt

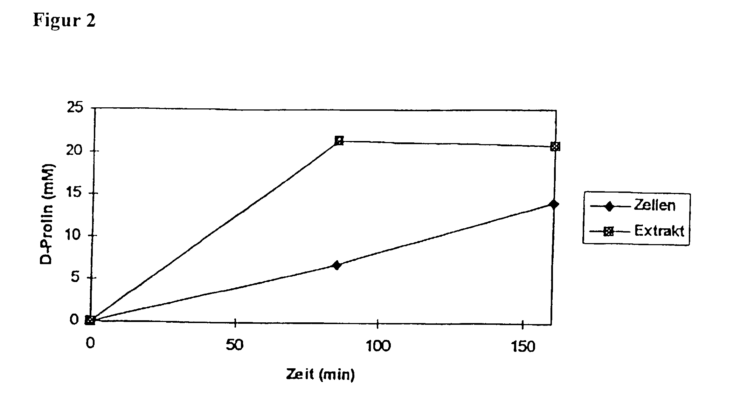

Die Zellen von Pseudomonas aeruginosa (DSM 10581) wurden durch die Frenchpresse (3mal; 120 MPa) aufgebrochen. Zu 1 ml zellfreien Extraktes wurde 1 ml 50 mM L-α-Chlor-δ-guanidinvaleriansäure in 100 mM Phosphatpuffer (pH 7 - 8) zugegeben und bei 37 °C inkubiert. Zum Vergleich wurde das Ganze auch mit ganzen Zellen durchgeführt. Proben wurden entnommen und mittels HPLC analysiert. Es zeigte sich, dass die Aktivität des zellfreien Extraktes mehr als doppelt so aktiv war wie die Aktivität der entsprechenden nicht aufgeschlossenen Zellen (Fig. 2).

**Tabelle 3**

| | |
|---|---|
| MgCl₂ x 6H₂O | 0,8 g/l |
| CaCl₂ x 6H₂O | 0,16 g/l |
| FeO₄ x 7H₂O | 20 mg/l |
| Spurenelemente ohne EDTA | 1 ml/l |
| Polypropylenglycol 2000 | 1 ml |
| Glucose | 10 g/l ("Glucose-feed" bis 30 g/l) |
| Na₂HPO₄ | 2 g/l |
| KH₂PO₄ | 1 g/l |
| NaCl | 2 g/l |
| Induktor: | α-Chlor-δ-guanidinvaleriansäure 10mM (Nachfüttern) |
| N-Quelle: | (NH₄)₂ SO₄, 3,1 g/l |

### c) Derivatisiserung von D-Prolin zu D-Z-Prolin

4,6 g wie oben isoliertes D-Prolin wurden in 20 ml H₂O gelöst. Bei einem konstanten pH von 11,5 - 12 (4N NaOH) wurde Chlorameisensäurebenzylester (Z-Cl) zugetropft. Insgesamt wurden 9,3 g Z-Cl zugetropft. Nach der Reaktion wurde mit HCl neutralisiert. Diese Lösung wurde mit Butylacetat extrahiert. Die organischen Phasen wurden verworfen. Durch weitere Zugabe von HCl zur wässrigen Phase wurde das pH auf 2 eingestellt, und abermals mit Butylacetat ausgeschüttelt. Die organischen Phasen wurden vereint und eingeengt. Das Produkt kristllisierte aus einer Lösung von 2,5 ml Ethylacetat und 1,5 ml Hexan aus. 3,6 g Z-D-Prolin wurden erhalten.

Schmelzpunkt: 68,5 °C
Gehalt: 97,86 %
[α_{D}²⁰] (c=2 in Eisessig) = +55,766°
[α₅₄₆²⁰] (c=2 in Eisessig) = +66,251°
ee (nach HPLC) 95,4%
¹H-NMR (400 MHz in CD₃OD); δ in ppm:
7,35 (m, 5H);
5,1 (m, 2H);
4,3 (m, 1H);
3,6 - 3,4 (m, 2H);
2,3 - 2,2 (m, 1H);
2,1 - 1,9 (m,3H)

### Beispiel 8:

### Enzymreinigung

Zellen von Pseudomonas aeruginosa DSM 10581 wurden wie in Beispiel 1 beschrieben angezüchtet. Diese wurden in der spätexponentiellen Wachstumsphase durch Zentrifugation geerntet, und mit 0,85% NaCl oder 30 - 200 mM Hepes-Puffer pH 7 - 9 gewaschen. Durch Frenchpresse (3mal; 120 MPa) wurden die Zellen aufgeschlossen. Der Zellextrakt wurde 30 Min bei 30'000 g abzentrifugiert. Der zellfreie Überstand wurde filtriert (0,4 µm) und über MonoQ-FPLC Säule (Pharmacia) gereinigt. Als mobile Phase wurde 10 mM Hepes Puffer pH 8 verwendet. Ein Gradient mit Na₂SO₄ wurde angelegt, um das Enzym von der Säule zu eluieren. Das Enzym eluierte bei einer Salzkonzentration von 120 mM von der Säule. 25 mM L-α-Chlor-δ-guanidinvaleriansäure wurden über Nacht mit dem angereicherten Enzym zu D-Prolin umgesetzt.

Die aktiven Fraktionen aus der MonoQ FPLC Reinigung wurden vereint und mit einer Phenylsepharosesäule (Pharmacia) weitergereinigt. Als mobile Phase wurde 50 mM Hepes-puffer pH 8, 1,7 M (NH₄)₂SO₄ verwendet. Die Hydrolase wurde mit 50 mM Hepes-Puffer pH 8 von der Säule eluiert. Das gereinigte Enzym transformierte 25 mM L-α-Chlor-δ-guanidinvaleriansäure innert 24 h vollständig zu L-α-Chlor-δ-guanidinvaleriansäure, welches in der Folge durch die Basizität im Medium zu D-Prolin cyclisierte.

### Beispiel 9:

### Enzymcharakterisierung

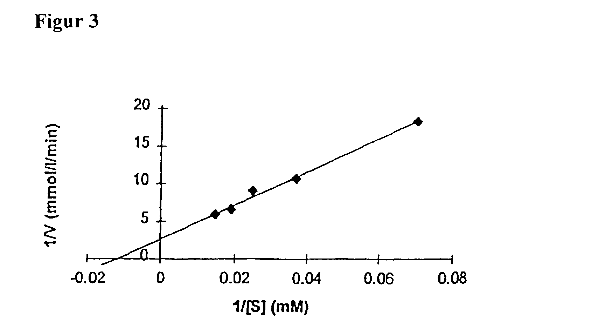

Die Enzymcharakterisierung wurde mit ganzen Zellen von Pseudomonas aeruginosa (DSM 10581) durchgeführt. Dabei betrug die optische Dichte der Zellsuspension OD₆₀₀ = 17. Der Einfluss der Temperatur wurde in 100 mM Phosphatpuffer (pH 7 - 8) bei einer Substratkonzentration von 25 mM L-α-Chlor-δ-guanidinvaleriansäure bestimmt. Dabei zeigte sich, dass bei 37 °C eine um 40% höhere Aktivität als bei 30 °C erhalten wurde. Der Einfluss des pH-Wertes wurde in gleichem Puffer, bei gleicher Substratkonzentration und bei einer Temperatur von 37 °C ermittelt. Gemessen wurde die Aktivität bei pH 7; pH 7,5; pH 8,0, pH 8,5. Dabei wurde festgestellt, dass das pH-Optimum bei pH 8,5 lag. Durch Einsatz von verschiedenen Konzentrationen an L-α-Chlor-δ-guanidinvaleriansäure wurde der K_{M}-Wert und Vₘₐₓ bei einer Temperatur von 37 °C, in 100 mM Phosphatpuffer (pH 8,5), bestimmt. Der K_{M}-Wert betrug 85,2 mmol / l und Vₘₐₓ 0,38 mmol / l / min (Fig. 3).

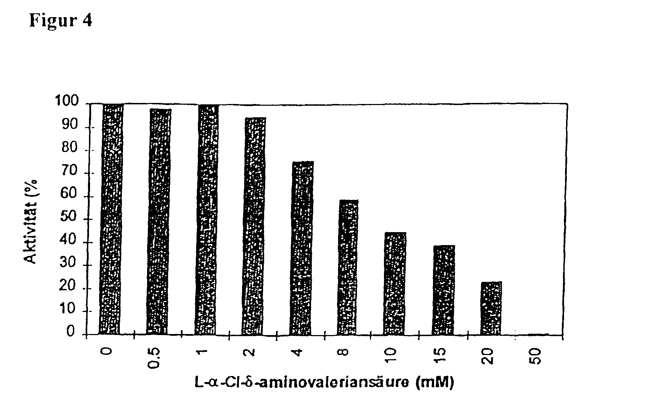

Durch Zugabe von verschiedenen Konzentrationen an L-α-Chlor-δ-aminovaleriansäure (hergestellt aus Ornithin analog zu Beispiel 6) konnte eine Hemmung nachgewiesen werden. Wie aus Fig. 4 ersichtlich, tritt bei einer Konzentration von 50 mM vollständige Hemmung auf.

## Patentansprüche

1. Mikroorganismen, dadurch gekennzeichnet, dass sie befähigt sind ein Guanidinvaleriansäurederivat ausgewählt aus den Verbindungen der allgemeinen Formel 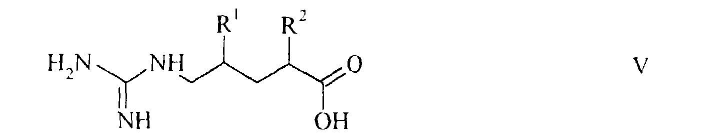 worin R¹ Wasserstoff oder Hydroxy und R² ein Halogenatom oder -NH₂ bedeutet, als einzige Stickstoffquelle zu verwerten, sowie Enzymextrakte daraus.

2. Mikroorganismen nach Anspruch 1 der Gattung Klebsiella, Pseudomonas, Agrobacterium oder Arthrobacter.

3. Mikroorganismen nach Anspruch 1 oder 2 der Spezies Klebsiella pneumoniae DSM 10593, Arthrobacter sp. DSM 10582, Agrobacterium radiobacter, Pseudomonas cepacia oder Pseudomonas aeruginosa DSM 10581, sowie deren funktionell aequivalente Varianten und Mutanten.

4. Enzym mit Amidinohydrolase-Aktivität, erhältlich aus Mikroorganismen nach einem der Ansprüche 1 bis 3 und befähigt, von Guanidinvaleriansäurederivaten, ausgewählt aus Verbindungen der allgemeinen Formel 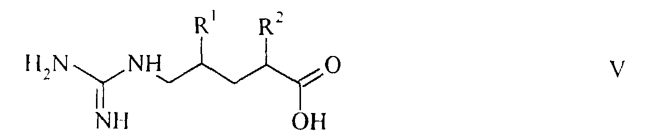 worin R¹ Wasserstoff oder Hydroxy und R² ein Halogenatom oder -NH₂ bedeutet, Harnstoff abzuspalten, sowie funktionell aequivalente Varianten und Mutanten davon.

5. Verfahren zur Herstellung von D-Prolinderivaten der allgemeinen Formel 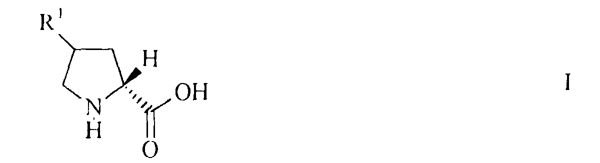 worin R¹ Wasserstoff oder Hydroxy bedeutet, dadurch gekennzeichnet, daß man ein L-Guanidinvaleriansäurederivat, oder dessen Salz, der allgemeinen Formel 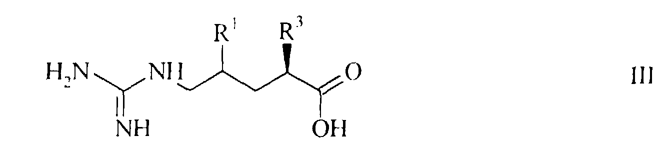 worin R¹ die genannte Bedeutung hat und R³ ein Halogenatom bedeutet, mit einem Mikroorganismus oder Enzymextrakt gemäss Anspruch 1 oder einer Amidinohydrolase gemäss Anspruch 4 zu einem L-Aminovaleriansäurederivat der allgemeinen Formel 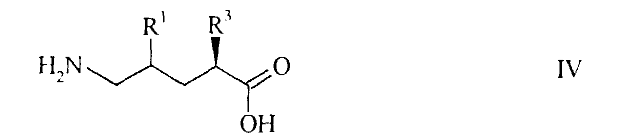 worin R¹ und R³ die genannte Bedeutung haben, hydrolysiert und dieses zum Endprodukt der Formel I cyclisiert.

6. Verfahren nach Anspruch 5, worin das L-Guanidinvaleriansäurederivat der Formel III, oder dessen Salz, in einer ersten Stufe durch Umsetzung eines L-Argininderivats, oder eines Salzes davon, der allgemeinen Formel 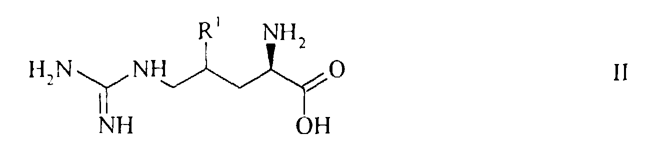 worin R¹ die genannte Bedeutung hat, hergestellt wird.

7. Verfahren nach Anspruch 6, worin die Umsetzung des L-Argininderivats oder eines seiner Salze bei einer Temperatur von -10 bis 100 °C durchgeführt wird.

8. Verfahren nach einem der Ansprüche 5 bis 7, worin die Hydrolyse des L-Guanidinvaleriansäurederivats der Formel III oder dessen Salzen mittels Mikroorganismen der Gattung Klebsiella, Pseudomonas, Agrobacterium oder Arthrobacter durchgeführt wird.

9. Verfahren nach Anspruch 8, worin die Hydrolyse mittels Mikroorganismen der Spezies Klebsiella pneumoniae DSM 10593, Arthrobacter sp. DSM 10582, Agrobacterium radiobacter, Pseuomonas cepacia oder mit Pseudomonas aeruginosa DSM 10581 oder mit deren funktionell aequivalenten Varianten und Mutanten durchgeführt wird.

10. Verfahren nach einem der Ansprüche 5 bis 9, worin die Hydrolyse bei einem pH-Wert von 5 bis 13 durchgeführt wird.

11. Verfahren nach einem der Ansprüche 5 bis 10, worin die Cyclisierung ohne Isolation des L-Aminovaleriansäurederivats der Formel IV durchgeführt wird.

## Claims

1. Microorganisms, characterised in that they are capable of metabolising a guanidinovaleric acid derivative selected from among the compounds of the general formula 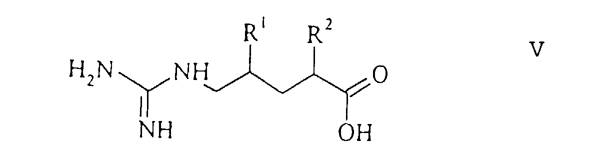 in which R¹ means hydrogen or hydroxy and R² means a halogen atom or -NH₂, as the sole source of nitrogen, together with enzyme extracts therefrom.

2. Microorganisms according to claim 1 of the genus *Klebsiella, Pseudomonas, Agrobacterium* or *Arthrobacter.*

3. Microorganisms according to claim 1 or 2 of the species *Klebsiella pneumoniae* DSM 10593, *Arthrobacter sp*. DSM 10582, *Agrobacterium radiobacter, Pseudomonas cepacia* or *Pseudomonas aeruginosa* DSM 10581, together with the functionally equivalent variants and mutants thereof.

4. Enzyme having amidinohydrolase activity obtainable from microorganisms according to one of claims 1 to 3 and capable of cleaving urea from guanidinovaleric acid derivatives, selected from among compounds of the general formula 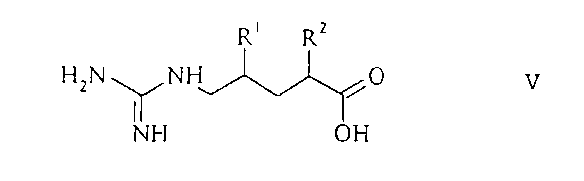 in which R¹ means hydrogen or hydroxy and R² means a halogen atom or -NH₂, together with functionally equivalent variants and mutants thereof.

5. Process for the production of D-proline derivatives of the general formula 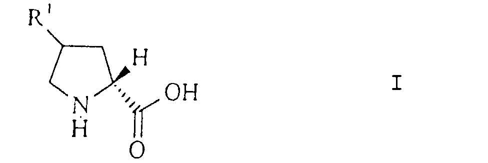 in which R¹ means hydrogen or hydroxy, characterised in that an L-guanidinovaleric acid derivative, or the salt thereof, of the general formula 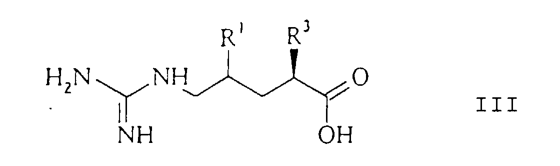 in which R¹ has the stated meaning and R³ means a halogen atom, is hydrolysed with a microorganism or enzyme extract according to claim 1 or an amidinohydrolase according to claim 4 to yield an L-aminovaleric acid derivative of the general formula 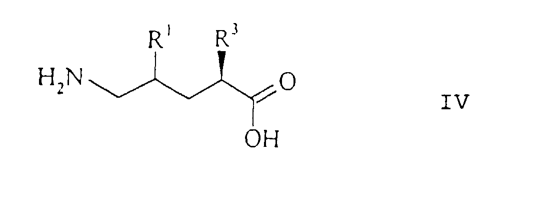 in which R¹ and R³ have the stated meaning and this is cyclised to yield the final product of the formula I.

6. Process according to claim 5, in which the L-guanidinovaleric acid derivative of the formula III, or the salt thereof, is produced in a first step by reacting an L-arginine derivative, or a salt thereof, of the general formula 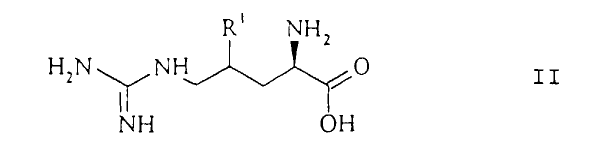 in which R¹ has the stated meaning.

7. Process according to claim 6, in which the reaction of the L-arginine derivative or of a salt thereof is performed at a temperature of -10 to 100°C.

8. Process according to one of claims 5 to 7, in which hydrolysis of the L-guanidinovaleric acid derivative of the formula III or of the salts thereof is performed by means of microorganisms of the genus *Klebsiella, Pseudomonas, Agrobacterium* or *Arthrobacter.*

9. Process according to claim 8, in which hydrolysis is performed by means of microorganisms of the species *Klebsiella pneumoniae* DSM 10593, Arthrobacter *sp.* DSM 10582, *Agrobacterium radiobacter, Pseudomonas cepacia* or with *Pseudomonas aeruginosa* DSM 10581 or with the functionally equivalent variants and mutants thereof.

10. Process according to one of claims 5 to 9, in which hydrolysis is performed at a pH value of 5 to 13.

11. Process according to one of claims 5 to 10, in which cyclisation is performed without isolating the L-aminovaleric acid derivative of the formula IV.

## Revendications

1. Micro-organismes caractérisés en ce qu'ils sont capables d'utiliser comme unique source d'azote un dérivé de l'acide guanidinevalérique choisi parmi les composés de formule générale 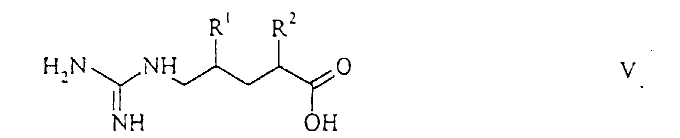 où R¹ représente l'hydrogène ou hydroxyle et R² représente un atome d'halogène ou -NH₂, ainsi que des extraits enzymatiques de ceux-ci.

2. Micro-organismes selon la revendication 1 du genre Klebsiella, Pseudomonas, Agrobacterium ou Arthrobacter.

3. Micro-organismes selon la revendication 1 ou 2 de l'espèce Klebsiella pneumoniae DSM 10593, Arthrobacter sp. DSM 10582, Agrobacterium radiobacter, Pseudomonas cepacia ou Pseudomonas aeruginosa DSM 10581, ainsi que leurs variantes et mutants fonctionnellement équivalents.

4. Enzyme à activité d'amidinohydrolase qui peut être obtenue à partir de micro-organismes selon l'une des revendications 1 à 3 et capable de cliver de l'urée à partir de dérivés de l'acide guanidinevalérique choisis parmi les composés de formule générale 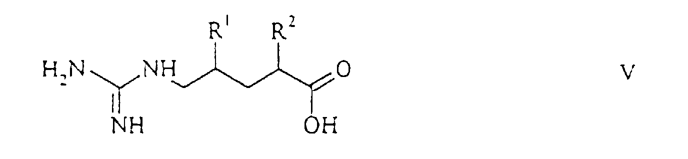 où R¹ représente l'hydrogène ou hydroxyle et R² représente un atome d'halogène ou -NH₂, ainsi que ses variantes et mutants fonctionnellement équivalents.

5. Procédé de préparation de dérivés de D-proline de formule générale 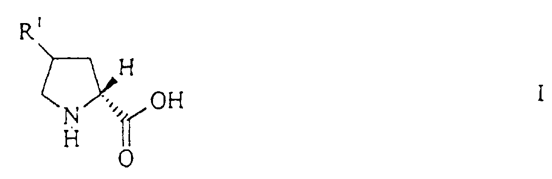 où R¹ représente l'hydrogène ou hydroxyle, caractérisé en ce que l'on hydrolyse un dérivé d'acide L-guanidinevalérique, ou son sel, de formule générale 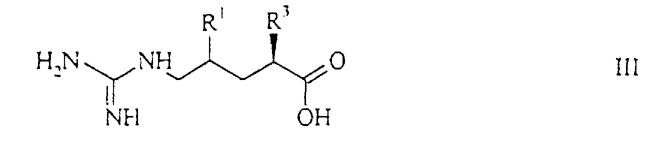 où R¹ a la signification citée et R³ représente un atome d'halogène, avec un micro-organisme ou extrait enzymatique selon la revendication 1 ou une amidinohydrolase selon la revendication 4 en un dérivé d'acide L-aminovalérique de formule générale 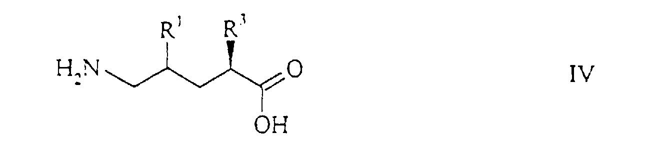 où R¹ et R³ ont la signification citée, et on cyclise celui-ci en le produit final de formule I.

6. Procédé selon la revendication 5 où le dérivé d'acide L-guanidinevalérique de formule III, ou son sel, est préparé dans une première étape par conversion d'un dérivé de L-arginine, ou d'un sel de celui-ci, de formule générale 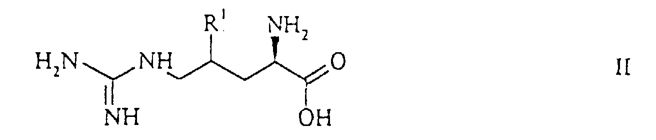 où R¹ a la signification citée.

7. Procédé selon la revendication 6 où la conversion du dérivé de L-arginine ou de l'un de ses sels est réalisée à une température de -10 à 100°C.

8. Procédé selon l'une des revendications 5 à 7 où l'hydrolyse du dérivé d'acide L-guanidinevalérique de formule III ou de son sel est réalisée au moyen de micro-organismes du genre Klebsiella, Pseudomonas, Agrobacterium ou Arthrobacter.

9. Procédé selon la revendication 8 où l'hydrolyse est réalisée au moyen de micro-organismes de l'espèce Klebsiella pneumoniae DSM 10593, Arthrobacter sp. DSM 10582, Agrobacterium radiobacter, Pseudomonas cepacia ou avec Pseudomonas aeruginosa DSM 10581 ou avec leurs variantes et mutants fonctionnellement équivalents.

10. Procédé selon l'une des revendications 5 à 9 où l'hydrolyse est réalisée à un pH de 5 à 13.

11. Procédé selon l'une des revendications 5 à 10 où la cyclisation est réalisée sans isolement du dérivé d'acide L-aminovalérique de formule IV.
